Europäisches Patentamt

(19) European Patent Office    (11) Numéro de publication: **0 214 068**

Office européen des brevets    **B1**

# (12) FASCICULE DE BREVET EUROPÉEN

(45) Date de publication du fascicule du brevet: **31.05.89**

(51) Int. Cl.⁴: **C 07 C 25/00**, C 07 C 17/22

(21) Numéro de dépôt: **86420197.5**

(22) Date de dépôt: **18.07.86**

(54) **Procédé de préparation de composés aromatiques fluorés.**

(30) Priorité: **19.08.85 FR 8511435**

(43) Date de publication de la demande:
**11.03.87 Bulletin 87/11**

(45) Mention de la délivrance du brevet:
**31.05.89 Bulletin 89/22**

(84) Etats contractants désignés:
**AT BE CH DE FR GB IT LI LU NL SE**

(56) Documents cité:
**US-A-2 939 766**

(73) Titulaire: **RHONE- POULENC CHIMIE, 25, quai Paul Doumer, F-92408 Courbevoie Cédex (FR)**

(72) Inventeur: **Seigneurin, Laurent, 3, avenue du Parc, F-30340 Salindres (FR)**

(74) Mandataire: **Le Pennec, Magali, RHONE- POULENC INTERSERVICES Service Brevets Chimie 25, Quai Paul Doumer, F-92408 Courbevoie Cédex (FR)**

EP 0 214 068 B1

## Description

La présente invention à pour objet un procédé de préparation de composés aromatiques fluorés; elle concerne plus particulièrement un procédé perfectionné de préparation de composés aromatiques fluorés par diazotation d'amines aromatiques dans de l'acide fluorhydrique puis décomposition in situ du fluorure de diazonium formé. La diazotation des amines aromatiques peut être réalisée dans l'acide fluorhydrique anhydre ou aqueux.

Un tel procédé est bien connu dans l'art antérieur. Il est bien connu également qu'il entraîne la perte d'une quantité importante d'acide fluorhydrique et la formation d'effluents difficiles à traiter compte tenu de la présence dans ces effluents de sodium.

En effet, la séparation par distillation de l'HF libre est limitée, d'une part par la combinaison HF, NaF qui est stable à la température de distillation et, d'autre part, par l'existence de l'azéotrope $H_2O$-HF.

Des tentatives ont été faites pour résoudre au moins partiellement ces inconvénients.

C'est ainsi que le brevet des Etats-Unis d'Amérique 2 939 766 décrit un procédé selon lequel on capte l'eau par addition de $SO_3$ anhydre puis on fait réagir l'acide sulfurique formé sur le mélange HF, NaF. Par distillation on peut récupérer l'HF, et simultanément $H_2SO_4$ réagit sur HF, NaF libérant de l'HF et formant du sulfate acide de soude.

On récupère bien l'HF mais on obtient des quantités importantes dc sulfate acide de soude et d'acide sulfurique qui sont très difficiles à éliminer et qui nécessitent un traitement particulier au plan de la protection de l'environnement.

Le sodium sous forme de sulfate de soude est perdu car irrécupérable dans des conditions industrielles et économiques satisfaisantes.

La présente invention pallie les inconvénients de l'art antérieur. Elle apporte un procédé de préparation de composés aromatiques fluorés par diazotation d'amines aromatiques permettant sans apport de composés additionnels étrangers au procédé, d'une part de récupérer sous forme d'HF anhydre la quasi-totalité de l'HF résiduaire et, d'autre part, de récupérer la totalité du sodium qui à été introduit au moment de la diazotation sous forme de nitrite de sodium. Un autre objectif atteint par un mode de mise en oeuvre particulièrement avantageux du procédé selon l'invention est de réutiliser dans le procédé lui-même la totalité de l'acide fluorhydrique récupéré et la grande majorité du sodium récupéré. C'est, sur le plan économique, un avantage très important.

La présente invention a donc pour objet un procédé de préparation de composés fluorés aromatiques de formule ArF dans laquelle Ar représente un radical aromatique, du type selon lequel on soumet une amine de formule $ArNH_2$ à une réaction de diazotation avec du nitrite de sodium dans de l'HF, le sel de diazonium formé étant ensuite décomposé thermiquement in situ, caractérisé en ce que ultérieurement,

- dans une première étape, on traite la couche minérale provenant de la décantation du mélange obtenu après diazotation et décomposition thermique, avec une quantité de fluorure de sodium telle que le rapport molaire du fluorure de sodium à l'HF libre présent dans le mélange est supérieur à 1;

- dans une deuxième étape, on sèche le mélange obtenu, éventuellement additionné de l'HF extrait des ef fluents gazeux de la réaction de diazotation, à une température telle que le fluorure acide de sodium HF, NaF n'est pas décomposé, et jusqu'à ce que l'eau soit totalement éliminée;

- et dans une troisième étape, on soumet le produit obtenu à une décomposition thermique à une température supérieure à 200° C, ce qui conduit à de l'acide fluorhydrique anhydre gazeux et du fluorure de sodium solide.

Selon un mode de réalisation particulièrement avantageux du procédé selon l'invention, l'acide fluorhydrique gazeux est, après condensation, recyclé à la réaction de diazotation et le fluorure de sodium est recyclé à la première étape après avoir soutiré la quantité correspondant au sodium introduit lors de la réaction de diazotation sous forme de nitrite de sodium.

La quantité de fluorure de sodium soutirée peut par exemple, après dissolution dans l'eau et filtration pour éliminer les goudrons présents, être utilisée sans traitement supplémentaire pour fabriquer des cryolithes.

Le procédé selon l'invention est, de préférence, conduit sous pression atmosphérique. Il peut également être mis en oeuvre sous pression supérieure à la pression atmosphérique.

Le procédé selon l'invention permet de récupérer sous forme d'HF et sous forme de NaF entre 95 et 99 % du fluor non utilisé lors de la réaction de diazotation.

On préfère, lors de la première étape, utiliser une quantité de fluorure de sodium telle que le rapport molaire du fluorure de sodium à l'HF libre présent dans le milieu est compris entre 1 et 1,5. Encore plus préférentiellement, ce rapport est égal à environ 1,2.

A la fin de cette première étape l'HF présent dans le milieu est totalement sous la forme combinée NaF, HF; on sera également en présence d'un excès de NaF et d'eau issue de la réaction de diazotation.

La réaction est exothermique. Pour éviter le départ d'HF gazeux il est préférable de maintenir la température à une valeur adéquate inférieure à 110° C et encore plus préférentiellement comprise entre 60 et 80° C, quand on est sous pression atmosphérique.

La température utilisée dans la deuxième étape n'est pas critique dans la mesure où la température du produit solide reste à tout moment inférieure à 110° C de façon à éviter la perte d'HF gazeux par décomposition de HF, NaF. Le séchage est poursuivi jusqu'à ce que l'eau soit totalement éliminée.

Dans le solide obtenu en fin de deuxième étape l'HF est sous forme HF, NaF. Est également présent le NaF en excès. La décomposition thermique de la troisième étape va permettre d'obtenir d'une part l'HF et d'autre part le NaF.

La température de mise en oeuvre dans cette troisième étape est de préférence comprise entre 250 et 400°C. Encore plus préférentiellement, on préfère opérer à 350°C environ.

Le procédé selon l'invention peut être mis en oeuvre en continu ou en discontinu.

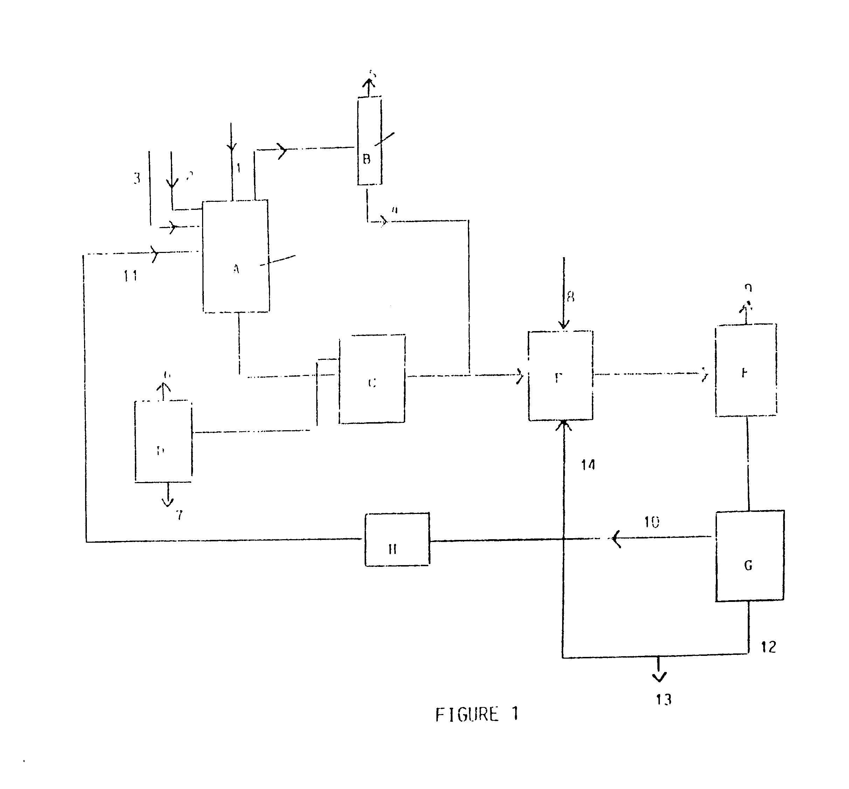

Le procédé va être maintenant plus complètement décrit à l'aide du schéma représenté en figure 1.

Le réacteur A dans lequel est effectuée la réaction de diazotation et de décomposition thermique est alimenté sucessivement en HF (1) en amine aromatique $Ar(NH_2)$ (2) et en nitrite de sodium (3). La réaction est conduite dans les conditions bien connues de l'art antérieur.

Lors de la réaction, un mélange gazeux d'HF et d'azote se dégage et est envoyé dans une colonne d'absorption B où l'on peut, par un lavage méthodique à l'eau, obtenir en 4 une solution d'HF de concentration égale à 20 - 40 %, l'azote s'échappant en 5.

Le mélange issu du réacteur A est envoyé dans un décanteur C où une couche organique et une couche minérale se séparent. La couche organique contient essentiellement le composé aromatique fluoré désiré, une petite partie des goudrons et une très faible quantité d'HF. La couche minérale est constituée d'HF, de NaF, HF d'eau et de goudrons insolubles et qui dans la plupart des cas se retrouvent en majeure partie dans la partie supérieure de la couche minérale près de l'interface organique.

La couche organique et la plus grande partie des goudrons rassemblés près de l'interface sont envoyés dans un bouilleur de colonne à distiller D où après neutralisation et distillation, le composé aromatique fluoré est obtenu en 6, les goudrons récupérés en 7 étant brûlés.

La couche minérale, éventuellement additionnée de la solution d'HF issue de la colonne B en 4 (ce qui augmente le taux de fluor récupéré) est mélangée dans un mélangeur E avec du NaF en quantité telle que le rapport molaire du NaF à l'HF libre soit supérieur à 1. On obtient immédiatement une poudre ou une pâte constituée de HF, NaF, d'eau et de NaF en excès. Ce mélange est envoyé dans un sécheur F où il est soumis à une température telle que la température du mélange soit inférieure à 110°C, jusqu'à ce que la totalité de l'eau soit éliminée en 9. Le produit sec est envoyé dans un four G où il est soumis à un traitement thermique à une température supérieure à 200°C. L'HF est récupéré en 10.

Le NaF est introduit en 8 si on utilise du NaF extérieur au procédé (et c'est nécessairement lors du démarrage) ou en 14 quand selon un mode de réalisation préférentiel on utilise le NaF issu de G. Bien que cela ne soit pratique, on peut utiliser, lors du démarrage, un composé donnant avec l'HF du NaF comme par exemple le carbonate de soude ou de la soude.

L'HF gazeux est condensé dans le condenseur H puis selon un mode de réalisation préférentiel réintroduit en 11 dans le réacteur A. Le NaF récupéré en 12 est de préférence, après soutirage en 13 d'une quantité équivalente à la quantité de sodium introduite dans le réacteur A sous forme de nitrite de sodium, recyclé en 14 dans le malaxeur E.

Si on le souhaite, le NaF récupéré en 13 contenant des goudrons est dissous dans l'eau puis filtré de façon à éliminer les goudrons fluorés, et récupéré sous une forme directement utilisable.

Le procédé selon l'invention est particulièrement bien adapté à la mise en oeuvre d'une amine de formule $ArNH_2$ ou $Ar(NH_2)_2$ dans laquelle Ar représente un radical naphtyle ou phényle comportant éventuellement au moins un substituant choisi parmi le groupe comprenant:
- les radicaux alkyle et alkoxy ayant de préférence de 1 à 6 atomes de carbone;
- les radicaux phényle et phénoxy;
- les radicaux phényle et phénoxy substitués par au moins un radical alkyle ou alkoxy ayant de préférence de 1 à 6 atomes de carbone ou halogéno;
- les radicaux halogéno;
- les radicaux CN, $CF_3$ et $OCF_3$.
- les radicaux -COOH et -OH

On peut citer comme exemples de telles amines l'aniline, les toluidines o, m, p, les chlorotoluidines.

Il est particulièrement intéressant de préparer selon le procédé de l'invention les dérivés suivants: le fluorobenzène, les fluorotoluènes o, m, p, les chlorofluorotoluènes.

## Exemple 1

Dans un réacteur de 750 litres agité à double enveloppe, on introduit 340 Kg d'HF anhydre et 160 kg d'aniline en maintenant la température par circulation de saumure dans la double enveloppe à une valeur inférieure à 10°C. On introduit ensuite 125 kg de nitrite de sodium de façon progressive de façon à ce que la température reste inférieure à 10°C.

Lorsque le nitrite de sodium à été complètement introduit, on fait monter la température jusqu'à 60°C en la contrôlant de façon à décomposer le fluorure de diazonium.

Les effluents gazeux contenant 50 kg d'azote et de vapeurs nitreuses et 55 kg d'HF sont séparés dans la colonne B.

Le milieu réactionnel est envoyé dans le décanteur C où la couche minérale et la couche organique se séparent par simple décantation.

La couche organique contient 133 kg de fluorobenzène, 5 kg environ de goudrons et de produits lourds.

La distillation de la couche organique et des goudrons collectés à l'interface après neutralisation permet d'obtenir 130 kg de fluorobenzène.

La couche minérale contient 180 kg d'HF libre, 36 kg d'HF lié à 76 kg de NaF, 60 kg d'$H_2O$ et quelques goudrons finement dispersés.

La couche minérale est envoyée dans un bac de stockage qu'alimente en continu (59 kg/h) un malaxeur horizontal E refroidi par double enveloppe, dans lequel on introduit simultanément du fluorure de sodium (75 kg/h) à une température de 60°C environ. Le temps de séjour est d'environ 30 minutes.

On envoie en continu dans un sécheur à plateaux superposés la poudre obtenue qui circule des plateaux supérieurs aux plateaux inférieurs à contre-courant d'un courant gazeux inerte chaud (130 - 150°C) la température du produit à la sortie du sécheur étant à tout moment inférieure à 110°C. L'eau évaporée (10 - 11 kg/h) s'échappe en 9 avec les gaz de chauffage.

Le solide est introduit en continu dans un four rotatif à chauffage externe et atteint en fin de séjour (2 h) une température de 350°C environ.

L'HF dégagé est refroidi dans un premier échangeur tubulaire à eau puis passe dans un condenseur à eau pour être refroidi à -20°C. On récupère ainsi 35 kg/h d'HF qui sont recyclés à l'alimentation.

Le NaF résultant est refroidi à 80°C environ dans un sécheur à lit fluide par de l'air à température ambiante, l'air réchauffé pouvant être envoyé en appoint du sécheur à plateaux précédent.

Il est ensuite dirigé par transport pneumatique à air dans le malaxeur (75 kg/h) après avoir soutiré 12 kg/h.

Il à donc été récupéré sous forme d'HF et sous forme de NaF 96 % du fluor disponible dans la partie minérale résultant de l'opération de décantation.

Ce taux de récupération est de 80 % par rapport au fluor non utilisé dans la réaction de diazotation. Il aurait pu être augmenté si on avait introduit dans le malaxeur les 55 kg d'HF récupérés par lavage méthodique des effluents gazeux issus du réacteur de diazotation.

**Exemple 2**

Orthofluorotoluène
Dans un réacteur agité de 6 litres, équipé des systèmes de refroidissement, de réchauffage et de dégazage nécessaires, on procède à une opération séquentielle de diazotation en introduisant sucessivement:
- 2700 g d'HF
- 1450 g d'orthotoluidine
puis 1000 g de $NO_2Na$.

On décompose le fluorure de diazonium formé, on obtient à la fin de la réaction deux couches: une, organique surnageante de 1050 g composée essentiellement d'orthofluorotoluène, et une couche inférieure minérale de 3650 g.

La phase inférieure décantée est récupérée, cette liqueur est très colorée et chargée en goudrons finement dispersés - densité 1,4. L'HF libre est dosé: 61,3 %.

1ère opération
Dans un malaxeur on introduit 140 g de cette liqueur (HF récupérable 86 g (4,3) moles). Puis, par fraction, 216 g de NaF (5,15 moles, 20 % en excès).

La température est maintenue à 50°C.

Après 20 minutes de malaxage on recueille 355 g de poudre que l'on sèche à 70°C. On obtient 335 g, le dosage par la soude indique une teneur en NaF-HF de 4,3 moles. Les 335 g sont placés dans une nacelle disposée dans un four tubulaire avec léger balayage de l'air.

On monte en 2 heures à 300°C puis 3 heures de 300 à 350°C. La perte de poids est de 78 g les 257 g de NaF contiennent encore 1,98 % d'HF, on a capté dans les barboteurs 74 g, 3,7 moles d'HF.

$$Rdt \frac{3,7}{4,3} = 86\%$$

2ème opération
230 g de NaF de l'opération 1 sont ajoutés à 140 g de la même liqueur suivant le procédé décrit dans l'opération n°1.

Le bilan s'établit alors:

HF récupérable: 0,22 mole HF contenu dans NaF
+ 4,30 moles (HF diazo)
4,52 moles

On obtient 365 g de poudre qui séchée à 70°C donne 340 g, et qui contiennent 4,45 moles de NaF-HF.

La décomposition menée dans le même appareillage que pour l'opération n°1 est conduite en montant la température en 2 h 30 à 375°C puis 2 heures à 375°C.

La perte de poids est de 88 g.

Les 252 g de NaF restant contiennent 1,75 % d'HF, on à récupéré 83 g d'HF dans les eaux soit 4,15 moles.

$$Rdt \frac{4,15}{4,52} = 91 \%$$

3ème opération
Pour 140 g de liqueur décantée on ajoute 230 g de NaF de l'opération n°2. On procède de la même manière qu'à l'opération n°2 la température de décomposition est encore augmentée. On monte en 2 heures 30 à 375°C puis 3 heures de 375 à 400°C.

Le rendement est de 92 %.

La teneur en HF de NaF décomposé est de 1,17 %.

4ème opération
Le rendement est de 93 %. Mêmes conditions qu'au n°3.

5ème opération à 10ème opération
Le rendement est de 93 - 94 %. Mêmes conditions qu'au n°3.

Le fluorure de sodium obtenu (240 g) est très peu coloré. Il donne après lessivage à l'eau chaude et filtration, une solution claire et incolore.

**Exemple 3**

Chlorofluorotoluène
Dans le même appareillage qu'à l'exemple 2 et suivant le même mode opératòire, on introduit:
- 1740 g de HF anhydre
- 1180 g de Amino 2 Chloro 6 Toluène
- 760 g de nitrite de sodium (NO₂Na).

Après décantation, on obtient une couche organique surnageante de 1050 g environ composée essentiellement de chlorofluorotoluène et une couche inférieure minérale de 2200 g environ.

Dans ce cas particulier, il y à nette accumulation de goudrons dans la phase minérale à l'interiace, le restant de la phase minérale est très peu chargé en matières en suspension.

La phase inférieure décantée est récupérée, cette liqueur est peu colorée, il n'y à pratiquement pas de goudrons en suspension, par analyse on obtient la composition suivante:

| | |
|---|---|
| HF libre | 59 % |
| NaF HF | 25 % |
| H₂O | 15 % |

La densite est de 1,42 et la teneur en fluor total de 70,5 %.

1ère opération
a) 200 g de cette phase minérale sont dilués avec 200 g d'eau dans un bécher en polyéthylène et, sous agitation, on introduit en 1 heure, 282 g d'une solution de soude à 41 %, la température est maintenue à 30 - 40°C, puis est ramenée sous agitation à 20 - 25°C en 30 minutes. On filtre, essore et sèche le solide à 60°C.

Poids obtenu 228 g à 97,8 % en NaF HF (dosage par NaOH) Total: 3,6 moles.

b) Décomposition du NaF HF obtenu.
Les 228 g sont mis à l'intérieur d'un four tubulaire avec un léger balayage d'air. On monte à 250°C en 45 minutes puis de 250 à 300°C en l'heure. On laisse à 300°C pendant 1 heure. On dose l'HF sorti dans des barboteurs d'eau: 71 g. Perte de poids: 73 g (HF théoriquement disponible: 72 g). Le rendement est de 98,6 %. On obtient 155 g de NaF.

2ème opération
A 100 g de la phase minérale décrite ci-dessus, diluée avec 100 cm³ d'eau, on ajoute en malaxant 155 g de NaF obtenu dans l'opération précédente.

Ce qui représente 110 % de l'HF libre et combiné sous forme NaF, HF, on maintient la température à 40 - 50°C.

On obtient 328 g de produit pâteux que l'on sèche à 60°C. 223 g de produit sec sont obtenus contenant 93,6 % de NaF, HF. On décompose comme précédemment, on obtient 66,5 g de HF soit 3,325 moles.

Soit $\frac{HF}{\text{fluor total introduit}}$ = $\frac{3,325}{3,71}$ = 89 %

Rendement de la décomposition: $\frac{3,325}{3,36}$ = 98,9 %

En enchaînant avec le NaF récupéré de l'opération précédente, on fait 5 opérations supplémentaires.

Le bilan sur six opérations est:

| | |
|---|---|
| Fluor total introduit: | 3,71 x 6 = 22,25 moles |
| HF récupéré: | 19,4 moles |
| Rendement 19,4 : 22,25: | 87,2 % |
| NaF récupéré: | 6 x 20 g = 2,85 moles |
| Rendement 2,85 : 22,25: | 12,8 % |

**Revendications**

1. Procédé de préparation de composés fluorés aromatiques de formule ArF dans laquelle Ar représente un radical armatique, de type selon lequel on soumet une amine de formule ArNH₂ à une réaction de diazotation avec du nitrite de sodium dans de l'HF, le sel de diazonium formé étant ensuite décomposé thermiquement, caractérisé en ce que, ultérieurement, dans une première étape, on traite la couche minérale provenant de la décantation du mélange obtenu après diazotation et décomposition thermique avec une quantité de fluorure de sodium telle, que le rapport molaire du fluorure de sodium à l'HF libre total présent dans le mélange est supérieur à 1, dans une deuxième étape, on sèche le mélange obtenu éventuellement additionné de l'HF extrait des effluents gazeux de la réaction de diazotation, à une température telle que le fluorure acide de sodium HF, NaF n'est pas décomposé et jusqu'à ce que l'eau soit éliminée et, dans une troisième étape, on soumet le produit obtenu à une décomposition thermique à une température supérieure à 200°C pour obtenir de l'HF gazeux et du fluorure de sodium.

2. Procédé selon la revendication 1, caractérisé en ce que l'HF gazeux obtenu à la troisième étape est recyclé, après condensation, à la réaction de diazotation.

3. Procédé selon l'une quelconque des revendications précédentes caractérisé en ce que le fluorure de sodium obtenu à la troisième étape est recyclé à la première étape après avoir soutiré la quantité correspondant au sodium introduit lors de la réaction de diazotation sous forme de nitrite de sodium.

4. Procédé selon l'une quelconque des revendications précédentes, caractérisé en ce que dans la première étape le rapport molaire du fluorure de sodium à l'HF libre total présent dans le mélange est compris entre 1 et 1,5.

5. Procédé selon la revendication 4, caractérisé en ce que le rapport molaire est d'environ 1,2.

6. Procédé selon l'une quelconque des revendications précédentes, caractérisé en ce que dans la première étape la température est inférieure à 110°C.

7. Procédé selon l'une quelconque des revendications précédentes caractérisé en ce que dans la deuxième étape la température du mélange est maintenue à une valeur inférieure à 110°C.

8. Procédé selon la revendication 7, caractérisé en ce que la température est comprise entre 60 et 80°C.

9. Procédé selon l'une quelconque des revendications précédentes, caractérisé en ce que, dans la troisième étape, la température est comprise entre 250 et 450°C.

10. Procédé selon la revendication 9, caractérisé en ce que la température est égale à environ 350°C.

**Patentansprüche**

1. Verfahren zur Herstellung aromatischer fluorierter Verbindungen der Formel ArF in der Ar einen aromatischen Rest darstellt, des Typs bei dem man ein Amin der Formel $ArNH_2$ einer Diazotierungsreaktion mit Natriumnitrit in HF unterwirft, und das gebildete Diazoniumsalz anschließend thermisch zersetzt wird,

dadurch gekennzeichnet, daß man später in einem ersten Schritt die mineralische Schicht, die bei der Dekantierung des nach Diazotierung und thermischer Zersetzung erhaltenen Gemischs entsteht, mit einer solchen Menge Natriumfluorid behandelt, daß das molare Verhältnis von Natriumfluorid zum gesamten freien HF, das in dem Gemisch anwesend ist, größer ist als 1, und daß man in einem zweiten Schritt das erhaltene Gemisch, dem gegebenenfalls aus den ausströmenden Gasen der Diazotierungsreaktion extrahiertes HF zugegeben wurde, bei einer solchen Temperatur trocknet, daß das saure Natriumfluorid HF, NaF nicht zersetzt wird, bis das Wasser eliminiert ist, und man in einem dritten Schritt das erhaltene Produkt einer thermischen Zersetzung bei einer Temperatur oberhalb von 200°C unterwirft, um gasförmiges HF und Natriumfluorid zu erhalten.

2. Verfahren nach Anspruch 1, dadurch gekennzeichnet, daß das im dritten Schritt erhaltene gasförmige HF nach Kondensation in die Diazotierungsreaktion zurückgeführt wird.

3. Verfahren nach einem der vorhergehenden Ansprüche, dadurch gekennzeichnet, daß das im dritten Schritt erhaltene Natriumfluorid in den ersten Schritt zurückgeführt wird, nachdem die Menge, die dem bei der Diazotierungsreaktion in Form von Natriumnitrit eingeführten Natrium entspricht, abgezogen wurde.

4. Verfahren nach einem der vorhergehenden Ansprüche, dadurch gekennzeichnet, daß im ersten Schritt das molare Verhältnis des Natriumfluorids zum gesamten in dem Gemisch anwesenden freien HF zwischen 1 und 1,5 liegt.

5. Verfahren nach Anspruch 4, dadurch gekennzeichnet, daß das molare Verhältnis ungefähr 1,2 beträgt.

6. Verfahren nach einem der vorhergehenden Ansprüche, dadurch gekennzeichnet, daß im ersten Schritt die Temperatur unterhalb von 110°C liegt.

7. Verfahren nach einem der vorhergehenden Ansprüche, dadurch gekennzeichnet, daß im zweiten Schritt die Temperatur des Gemischs auf einem Wert unterhalb von 100°C gehalten wird.

8. Verfahren nach Anspruch 7, dadurch gekennzeichnet, daß die Temperatur zwischen 60 und 80°C liegt.

9. Verfahren nach einem der vorhergehenden Ansprüche, dadurch gekennzeichnet, daß im dritten Schritt die Temperatur zwischen 250°C und 450°C liegt.

10. Verfahren nach Anspruch 9, dadurch gekennzeichnet, daß die Temperatur etwa 350°C beträgt.

**Claims**

1. Process for preparing aromatic fluorinated compounds of formula ArF, in which Ar denotes an aromatic radical, of the type according to which an amine of formula $ArNH_2$ is subjected to a diazotization reaction with sodium nitrite in HF, the diazonium salt formed then being thermally decomposed, characterized in that, subsequently, in a first stage, the inorganic layer derived from the decantation of the mixture obtained after diazotization and thermal decomposition is treated with an amount of sodium fluoride such that the mol ratio of sodium fluoride to the total free HF present in the mixture is greater than 1, in a second stage, the mixture obtained, with the optional addition of the HF extracted from the gaseous effluents of the diazotization reaction, is dried at a temperature such that the acid sodium fluoride HF. NaF is not decomposed, and until the water is removed and, in a third stage, the product obtained is subjected to thermal decomposition at a temperature above 200°C, to obtain sodium fluoride and gaseous HF.

2. Process according to claim 1, characterized in that the gaseous HF obtained in the third stage is recycled, after condensation, to the diazotization reaction.

3. Process according to any one of the preceding claims, characterized in that the sodium fluoride obtained in the third stage is recycled to the first stage after withdrawing the amount corresponding to the sodium introduced in the form of sodium nitrite during the diazotization reaction.

4. Process according to any one of the preceding claims, characterized in that, in the first stage, the mol ratio of sodium fluoride to the total free HF present in the mixture is between 1 and 1.5.

5. Process according to claim 4, characterized in that the mol ratio is approximately 1.2.

6. Process according to any one of the preceding claims, characterized in that, in the first stage, the temperature is below 110° C.

7. Process according to any one of the preceding claims, characterized in that, in the second stage, the temperature of the mixture is maintained at a value below 110° C.

8. Process according to claim 7, characterized in that the temperature is between 60 and 80° C.

9. Process according to any one of the preceding claims, characterized in that, in the third stage, the temperature is between 250 and 450° C.

10. Process according to claim 9, characterized in that the temperature is equal to approximately 350° C.

FIGURE 1